# NOUVEAU FASCICULE DE BREVET EUROPEEN

(11) **EP 0 586 484 B2**
(45) Date de publication et mention de la décision concernant l'opposition: **14.01.1998**
(45) Mention de la délivrance du brevet: 01.03.1995
(21) Numéro de dépôt: 92911392.6
(22) Date de dépôt: 02.06.1992
(51) Int. Cl.: A61K 7/00, A61K 7/42, A61K 7/06

(54) **UTILISATION DE NANOPIGMENTS D'OXYDES METALLIQUES POUR PROTEGER LA KERATINE DES CHEVEUX CONTRE LES AGRESSIONS ATMOSPHERIQUES, NOTAMMENT LA LUMIERE, PROCEDE DE PROTECTION DES CHEVEUX ET COMPOSITION GELIFIEE UTILISANT CES NANOPIGMENTS**
VERWENDUNG VON METALLOXIDMANOPIGMENTEN ZUM SCHUTZ VON HAARKERATIN VOR ÄUSSEREN EINFLÜSSEN, INSBESONDERE SONNENLICHT, VERFAHREN ZUM SCHUTZ DES HAARES UND GELZUSAMMENSETZUNG UNTER VERWENDUNG DIESER NANOPIGMENTE
USE OF METALLIC OXIDE NANOPIGMENTS FOR PROTECTING HAIR KERATIN AGAINST EXTERNAL INFLUENCES, ESPECIALLY SUNLIGHT, PROCESS FOR PROTECTING HAIR AND GELIFIED COMPOSITION USING SAID NANOPIGMENTS

(30) Priorité: 04.06.1991 FR 9106745
(43) Date de publication de la demande: 16.03.1994
(73) Titulaire: L'OREAL, 75008 Paris (FR)
(72) Inventeur: DUPUIS, Christine, F-75018 Paris (FR); DUBIEF, Claude, F-78150 Le Chesnay (FR)
(74) Mandataire: Casalonga, Axel
(86) Numéro de dépôt international: FR9200486
(87) Numéro de publication internationale: WO9221315

(56) Documents cités:
- EP-A- 0 200 839
- WO-A-90/06103
- WO-A-90/11067
- GB-A- 2 184 356
- PATENT ABSTRACTS OF JAPAN vol. 13, no. 336 (C-623)(3684) 27 Juillet 1989
- SEIFEN, öLE, FETTE, WACHSE vol. 113, no. 20, 10 Décembre 1987, AUGSBURG pages 765 - 771; WOLF-DIETER GRIEBLER: 'Titandioxid - ein anorganischer Rohstoff in der Kosmetik' 2.1.3 : Transparantes TiO2 als UV-Absorber
- COSMETICS & TOILETRIES vol. 105, Février 1990, pages 53 - 64; MITCHELL L. SCHLOSSMAN: 'Treated Pigments. New Ways to Impart Color on the Skin' cité dans la demande
- PATENT ABSTRACTS OF JAPAN vol. 13, no. 461 (C-645)(3809) 18 Octobre 1989
- W.D. Griebler, Siefen-Ole-Fette-Wachse-113.Jg, N 20, 1987
- "Keratin et Keratinization" de E.H. Mercer, 1961, p. 9, 27, 29-32, 64-65, 161 et 245-249

## Description

La présente invention a pour objet l'utilisation de nanopigments d'oxydes métalliques en tant qu'agents de protection de la kératine des cheveux contre les agressions atmosphériques, et en particulier la lumière, ainsi qu'un procédé de protection des cheveux et une composition sous forme de gel utilisant ces nanopigments d'oxydes métalliques.

On sait depuis longtemps que la lumière agresse la kératine des cheveux. De nombreuses publications divulguent que la lumière naturelle détruit certains aminoacides des cheveux et qu'en altérant la fibre capillaire, elle en diminue les propriétés mécaniques comme la résistance à la traction, la charge de rupture et l'élasticité.

La résistance à la traction peut être mesurée par le palier à 15% d'extension. Le palier à 15% d'extension est la force qu'il faut appliquer à un cheveu mouillé d'une longueur donnée pour l'allonger de façon permanente de 15%. Plus cette force est élevée, plus le cheveu est élastique et résistant.

Pour lutter contre l'agression de la kératine des cheveux par la lumière, on a déjà proposé d'utiliser certains polyaminoamides (brevet français n° 2 599 968) ou certaines substances susceptibles de filtrer les radiations lumineuses, coninie l'acide 2-hydroxy-4-méthoxy benzophénone-5-sulfonique ou ses sels (brevet français n° 2 627 085) ou l'acide 4-(2-oxo-3-bornylidène méthyl) benzène sulfonique ou ses sels (demande de brevet européen n° 0329032).

La demanderesse vient de découvrir de façon tout à fait surprenante, que les nanopigments d'oxydes de métaux choisis parmi le titane, le zinc, le cérium et le zirconium, de diamètre moyen inférieur à 100 nanomètres, et plus particulièrement compris entre 5 et 50 nanomètres, pouvaient préserver les propriétés mécaniques des cheveux, et notamment leur résistance à la traction et leur élasticité, contre la dégradation par la lumière. Cette propriété a pu être mise en évidence par exposition en lumière naturelle (milieu ensoleillé) et en lumière artificielle (émetteur au xénon d'un appareil de vieillissement accéléré du type SUNTEST HANAU).

La présente invention a donc pour objet l'utilisation de nanopigments d'oxydes de métaux choisis parmi le titane, le zinc, le cérium et le zirconium ou leurs mélanges, en une quantité comprise entre 0,2 et 2% en poids en tant qu'agents de protection des propriétés mécaniques des cheveux, et essentiellement de la résistance-à la traction, contre la dégradation provoquée par les agressions atmosphériques, et en particulier par la lumière.

On entend par "nanopigments" des pigments de diamètre moyen inférieur à 100 nanomètres et de préférence compris entre 5 et 50 nm.

Ces oxydes métalliques peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries - 1990 - Vol. 105 - Fév.1990 - p. 53-64, tels que des aminoacides, de la cire d'abeille, des acides gras, des alcools gras, des tensio-actifs anioniques, des lécithines, des sels de sodium, potassium, zinc, fer ou aluminium, d'acides gras, de l'hexamétaphosphate de sodium, des alcoxydes métalliques (de titane ou d'aluminium), du polyéthylène, des silicones, des protéines (collagène. élastine), des alcanolamines, des oxydes de silicium, ou d'autres oxydes métalliques.

Les pigments enrobés sont plus particulièrement des oxydes de titane enrobés :
- de silice tel que le produit "SUNVEIL" de la société IKEDA,
- de silice et d'oxyde de fer tel que le produit "SUNVElL F" de la société IKEDA,
- de silice et d'alumine tels que les produits "MT 500 SA" et "MT 100 SA" de la société TAYCA et "TIOVEIL" de la société TIOXIDE,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tel que le produit "MT 100 T" de la société TAYCA,
- d'alumine et de laurate d'aluminium, tel que le produit "MIT 100 S" de la société TAYCA,
- d'oxyde de fer et de stéarate de fer tel que le produit "MIT 100 F" de la société TAYCA,
- d'oxyde de zinc et de stéarate de zinc tel que le produit "BR 351" de la société TAYCA,
- de silice, d'alumine et de silicone tels que les produits "MT 600 SAS" et "MT 500 SAS" de la société TAYCA,
- de silice, d'alumine, de stéarate d'aluminium et de silicone tel que le produit "STT-30-DS" de la société TITAN KOGYO.
- d'alumine et de silicone tel que le produit "TIPAQUE TTO-55 (S)" de la société ISHIHARA.
- de triéthanolamine tel que le produit "STT-65-S" de la société TITAN KOGYO,
- d'acide stéarique tel que le produit "TIPAOUE TTO-55 (C) de la société ISHIHARA.
- d'hexamétaphosphate de sodium tel que le produit "MT 150 W" de la société TAYCA.

On peut également citer les mélanges d'oxydes métalliques, dont le mélange équipondéral d'oxyde de titane et d'oxyde de cérium enrobés de silice, vendu par la société IKEDA sous la dénomination "SUNVEIL A".

Les oxydes de titane non enrobés sont par exemple vendus par la société TAYCA sous les dénominations commerciales "MT 500 B" ou "MT 600 B", par la société DEGUSSA sous la dénomination "P 25", par la société WACKHERR sous la dénomination "Oxyde de titane transparent PW" et par la société MIYOSHI KASEI sous la dénomination "UFTR".

Les oxydes de zinc non enrobés sont par exemple vendus par la société SUMITOMO sous la dénomination "ULTRA FINE ZINC OXIDE POWDER", par la société PRESPERSE sous la dénomination "FINEX 25", ou par la société IKEDA sous la dénomination "MZO-25".

L'oxyde de cérium non enrobé est par exemple vendu sous la dénomination "COLLOIDAL CERIUM OXIDE" par la société RHONE POULENC.

Ces pigments sont utilisables en mélange également.

Les pigments d'oxyde de titane, enrobés ou non enrobés, sont particulièrement préférés pour être utilisés selon l'invention.

Selon l'invention, on utilise les nanopigments pour préserver les propriétés mécaniques des cheveux des agressions par la lumière en une quantité comprise entre 0,2 et 2% en poids, dans un support cosmétique non rincé.

Selon un mode de réalisation préféré, on utilise ces nanopigments dans un support cosmétique gélifié. Les agents gélifiants utilisables à ce titre sont choisis parmi :
- les acides polyacryliques réticulés par un agent polyfonctionnel tels que plus particulièrement les produits vendus sous la dénomination "CARBOPOL"par la société GOODRICH tels que les Carbopol 910, 934, 934, P.940, 941, 1342;
- les dispersions aqueuses de copolymère d'acrylate d'ammonium/acrylamide (95/5 en poids) réticulé par un agent de réticulation à polyinsaturation oléfinique, dispersé dans une émulsion eau-dans-l'huile constituée par 30% en poids dudit copolymère, 25% en poids de paraffine, 4% de mélange de stéarate de sorbitan et de dérivé éthoxylé hydrophile, 41% en poids d'eau; une telle émulsion est commercialisée sous le non, de PAS 5161 par la société HOECHST;
- les gels résultant de l'interaction ionique d'un polymère cationique constitué par un copolymère de cellulose ou d'un dérivé de cellulose greffé avec un sel de monomère hydrosoluble d'ammonium quaternaire et d'un polymère anionique carboxylique ayant une viscosité capillaire absolue dans le diméthylformamide ou le méthanol, à une concentration de 5% et à 30°C, inférieure ou égale à 30 x 10⁻³ Pa.s, le gel lui-même ayant une viscosité Epprecht-Drage, module 3, en solution à 1% dans l'eau à 25°C, supérieure ou égale à 0,50 Pa.s.

Ces gels sont décrits dans le brevet français n° 2 598 611.

Parmi ceux-ci, on préfère utiliser le produit résultant de l'interaction ionique d'un copolymère d'hydroxyéthylcellulose greffé par voie radicalaire par du chlorure de diallyl diméthylammonium tel que le produit vendu sous la dénomination "CELQUAT L 200" par la société National Starch avec un copolymère de l'acide méthacrylique et du méthacrylate de méthyle ayant une viscosité capillaire mesurée en solution dans le diméthylformamide à la concentration de 5% et à 30°C, de l'ordre de 15.10⁻³ Pa.s.

A titre particulièrement préféré, on utilise le produit résultant de l'interaction ionique du copolymère d'hydroxyéthylcellulose susmentionné avec un copolymère d'acide méthacrylique et d'acrylate d'éthyle réticulé vendu sous les dénominations VISCOATEX 538, 46 ou 50 par la société COATEX.

Dans la présente invention sont particulièrement préférés les oxydes de titane non enrobés de granulométrie 15 à 40 nanomètres utilisés dans un support cosmétique gélifié par le copolymère d'hydroxyéthylcellulose ci-dessus mentionné associé à un copolymère d'acide méthacrylique et de méthacrylate de méthyle ou de préférence à un copolymère d'acide méthacrylique et d'acrylate d'éthyle réticulé vendu sous les dénominations VISCOATEX 538, 46 ou 50 par la société COATEX.

La présente invention a également pour objet une composition cosmétique pour cheveux sous forme de gel.

Les compositions cosmétiques pour cheveux conformes à l'invention et destinées à protéger les cheveux contre la dégradation par la lumière contiennent, à titre de composé actif, au moins un nanopigment d'oxydes de métaux choisis parmi le titane, le zinc, le cérium et le zirconium. Elles se présentent sous forme de gels et constituent des compositions non rincées pour le coiffage, la mise en plis ou le brushing.

Les agents gélifiants mentionnés ci-dessus sont utilisés dans les compositions selon l'invention dans des proportions comprises entre 0,1 et 30% en poids, et de préférence entre 0,2 et 10% en poids, par rapport au poids total de la composition.

Les compositions cosmétiques pour cheveux selon l'invention ont un pH compris entre 2 et 11 et de préférence entre 3 et 9.

Les compositions cosmétiques selon l'invention peuvent également renfermer des agents cosmétiques bien connus dans la technique sous réserve qu'ils n'altèrent pas eux-mêmes les propriétés mécaniques de la kératine des cheveux.

Les adjuvants ou agents cosmétiques généralement présents dans les compositions cosmétiques selon l'invention, sont par exemple des polymères non-ioniques, anioniques ou cationiques, des conservateurs, des huiles, des agents de régulation de pH, des cires, des agents antigras, des agents séquestrants, des parfums, des colorants, des agents tensioactifs cationiques, des protéines, des silicones ou des solvants organiques.

Les polymères particulièrement utilisés dans ces compositions pour apporter de la tenue aux cheveux sont des polymères non-ioniques comme les polyvinylpyrrolidones, les copolymères de polyvinylpyrrolidone et d'acétate de vinyle, ou encore des polymères anioniques comme les copolymères d'acétate de vinyle et d'un acide carboxylique insaturé tel que l'acide crotonique, les copolymères résultant de la copolymérisation d'acétate de vinyle, d'acide crotonique et d'un ester acrylique ou méthacrylique, les copolymères résultant de la copolymérisation d'acétate de vinyle, d'un éther alcoylvinylique et d'un acide carboxylique insaturé et les copolymères résultant de la copolymérisation d'acétate de vinyle, d'acide crotonique et d'un ester vinylique d'un acide à longue chaîne carbonée ou encore d'un ester allylique ou méthallylique d'un acide à longue chaîne carbonée. Ces polymères sont utilisés dans des concentrations comprises entre 0.1 et 5% en poids par rapport au poids total de la composition.

Ces compositions se présentent sous forme aqueuse mais peuvent contenir d'autres solvants acceptables sur le plan cosmétique tels que par exemple des alcools inférieurs comme l'éthanol ou l'isopropanol, du glycérol, des glycols ou éthers de glycol comme l'éther monobutylique de l'éthylèneglycol, le propylèneglycol, le monoéthyléther et le monométhyléther du diéthylèneglycol, et dans des proportions n'affectant pas la formation du gel.

La présente invention vise également un procédé de protection de la kératine des cheveux contre les agressions atmosphériques, et en particulier contre la lumière, consistant à appliquer sur les cheveux une quantité efficace d'au moins un nanopigment d'oxydes de métaux choisis parmi le titane, le zinc, le cérium et le zirconium, dans un support cosmétique, cette application n'étant pas suivie d'un rinçage.

Les exemples suivants illustrent l'invention sans pour autant la limiter.

### EXEMPLE 1

On prépare un gel de coiffage de composition suivante :

| | |
|---|---|
| - Dioxyde de titane (diamètre : 15 à 40 nanomètres) vendu sous la dénomination "P 25" par la société DEGUSSA | 1 g |
| - Copolymère d'acide méthacrylique et de méthacrylate de méthyle (50/50) | 0,7 g |
| - Copolymère d'hydroxyéthylcellulose et de chlorure de diallyl diméthyl ammonium vendu sous la dénomination "CELQUAT L 200" par la société NATIONAL STARCH | 0,7 g |
| - Polymère cationique siliconé vendu à la concentration de 35% de MA sous la dénomination "Emulsion cationique DC929" par la société DOW CORNING | 0,35 g MA |
| - Copolymère de vinylpyrrolidone et de méthacrylate de diméthylaminoéthyle quaternisé vendu à 50% de MA sous la dénomination "GAFQUAT 734" par la société GAF | 0,1 g MA |
| - 2-Amino-2-méthyl-propanol-1 qs pH 7.5 | |
| - Alcool éthylique | 8,6 g |
| - Parfum, conservateur, colorant qs | |
| - Eau qsp | 100 g |

### EXEMPLE 2

On prépare un gel de coiffage de composition suivante :

| | |
|---|---|
| - Dioxyde de titane (diamètre : 15 à 40 nanomètres) vendu sous la dénomination "P 25" par la société DEGUSSA | 1 g |
| - Acide polyacrylique réticulé vendu sous la dénomnation "CARBOPOL 940" par la société GOODRICH | 0,45 g |
| - Copolymère de vinylpyrrolidone et d'acétate de vinyle (65/35) vendu sous la dénomination "PVP/VA S 630" par la société GAF | 1 g MA |
| - Alcool éthylique | 17,2 g |
| - Triéthanolamine qs pH 7.5 | |
| - Eau qsp | 100 g |

### EXEMPLE 3

On prépare un gel de coiffage de composition suivante :

| | |
|---|---|
| - Oxyde de zinc (diamètre : 5 à 15 nanomètres) vendu par la société SUMITOMO sous la dénomination "ULTRA FINE ZINC OXIDE POWDER" | 1 g |
| - Copolymère d'acide méthacrylique et de méthacrylate de méthyle (50/50) | 0,7 g |
| - Copolymère d'hydroxyéthylcellulose et de chlorure de diallyl diméthylammonium vendu sous la dénomination "CELQUAT L 200" par la société NATIONAL STARCH | 0,7 g |
| - Polymère cationique siliconé vendu à la concentration de 35% de MA sous la dénomination "Emulsion cationique DC929" par la société DOW CORNING | 0.35 g MA |
| - Copolymère de vinylpyrrolidone et de méthacrylate de diméthylaminoéthyle quaternisé vendu à 50% de MA sous la dénomination "GAFQUAT 734" par la société GAF | 0,1 g MA |
| - 2-Amino-2-méthyl-propanol-1 qs pH 7.5 | |
| - Alcool éthylique | 8.6 g |
| - Parfum, conservateur, colorant qs | |
| - Eau qsp | 100 g |

### EXEMPLE 4

On prépare un gel de coiffage de composition suivante :

| | |
|---|---|
| - Dioxyde de titane (diamètre : 15 à 40 nanomètres) vendu sous la dénomination "P 25" par la société DEGUSSA | 1 g |
| - Copolymère d'acide méthacrylique et d'acrylate d'éthyle réticulé vendu à 30% de MA sous la dénomination VISCOATEX 50 par la société COATEX | 2 gMA |
| - Copolymère d'hydroxyéthylcellulose et de chlorure de diallyl diméthylammonium vendu sous la dénomination "CELQUAT L 200" par la société NATIONAL STARCH | 1 g |
| - Polymère cationique siliconé vendu à la concentration de 35% de MA sous la dénomination "Emulsion cationique DC929" par la société DOW CORNING | 0,3 g MA |
| - Copolymère de vinylpyrrolidone et d'acétate de vinyle (65/35) vendu sous la dénomination "PVP/VA S630" par la société GAF 0,1 g MA | 1 g MA |
| - 2-Amino-2-méthyl-propanol-1 qs pH 7.5 | |
| - Parfum, conservateur qs | |
| - Eau qsp | 100 g |

## Revendications

1. Utilisation en une quantité comprise entre 0,2 et 2% en poids. de nanopigments d'oxydes de métaux choisis parmi le titane, le zinc, le cérium, et le zirconium ou leurs mélanges, de diamètre moyen inférieur à 100 nanomètres, en tant qu'agents de protection des propriétés mécaniques des cheveux contre les agressions atmosphériques, et en particulier contre la lumière.

2. Utilisation selon la revendication 1 de nanopigments de diamètre compris entre 5 et 50 nanomètres.

3. Utilisation selon la revendication 1 ou 2 de nanopigments d'oxydes de titane.

4. Utilisation de nanopigments d'oxydes métalliques non enrobés selon l'une quelconque des revendications 1 à 3.

5. Utilisation de nanopigments d'oxydes métalliques enrobés selon l'une quelconque des revendications 1 à 3, ces pigments ayant subi un ou plusieurs traitements de surface de nature chimique, électronique, mécanochimique et/ou mécanique avec des composés choisis parmi les aminoacides, la cire d'abeille, les acides gras, les alcools gras, les tensio-actifs anioniques, les lécithines, les sels de sodium, potassium, zinc, fer ou aluminium d'acides gras, l'hexamétaphosphate de sodium, les alcoxydes métalliques, le polyéthylène, les silicones, les protéines, les alcanolamines et les oxydes de silicium.

6. Utilisation de nanopigments d'oxydes métalliques enrobés selon la revendication 5, choisis parmi les oxydes de titane enrobés de silice, de silice et d'oxyde de fer, de silice et d'alumine, d'alumine, d'alumine et de stéarate d'aluminium d'alumine et de laurate d'aluminium, d'oxyde de fer et de stéarate de fer, d'oxyde de zinc et de stéarate de zinc, de silice et d'alumine et de silicone, de silice et d'alumine et de stéarate d'aluminium et de silicone, d'alumine et de silicone, de triéthanolamine, d'acide stéarique ou d'hexamétaphosphate de sodium.

7. Utilisation de nanopigments d'oxydes de titane et de cérium enrobés de silice selon la revendication 5.

8. Procédé de protection des propriétés mécaniques des cheveux, et notamment de leur résistance à la traction et de leur élasticité, contre les agressions atmosphériques et en particulier contre la lumière, caractérisé par le fait qu'il consiste à appliquer sur les cheveux une quantité efficace pour protéger leurs propriétés mécaniques, comprise entre 0,2 et 2% en poids, d'au moins un nanopigment de diamètre moyen inférieur à 100 nanomètres d'oxydes de métaux choisis parmi le titane, le zinc, le cérium et le zirconium, dans un support cosmétique, cette application n'étant pas suivie d'un rinçage.

9. Procédé selon la revendication 8, caractérisé par le fait qu'on utilise au moins un nanopigment de diamètre compris entre 5 et 50 nanomètres.

10. Procédé selon la revendication 8 ou 9, caractérisé par le fait qu'on utilise un nanopigment d'oxyde de titane.

11. Procédé selon l'une quelconque des revendications 8 à 10, caractérisé par le fait qu'on utilise au moins un nanopigment d'oxydes métalliques non enrobés.

12. Procédé selon l'une quelconque des revendications 8 à 10, caractérisé par le fait qu'on utilise au moins un nanopigment d'oxydes métalliques enrobés ayant subi un ou plusieurs traitements de surface de nature chimique, électronique, mécanochimique et/ou mécanique avec des composés choisis parmi les aminoacides, la cire d'abeille, les acides gras, les alcools gras, les tensio-actifs anioniques, les lécithines, les sels de sodium, potassium, zinc, fer ou aluminium d'acides gras, l'hexamétaphosphate de sodium, les alcoxydes métalliques, le polyéthylène, les silicones, les protéines, les alcanolamines et les oxydes de silicium.

13. Procédé selon la revendication 12, caractérisé par le fait qu'on utilise au moins un nanopigment d'oxydes de titane enrobés de silice, d'alumine, de triéthanolamine, d'acide stéarique, d'hexamétaphosphate de sodium, de silice et d'oxyde de fer, de silice et d'alumine, d'alumine et de stéarate d'aluminium, d'alumine et de laurate d'aluminium, d'oxyde de fer et de stéarate de fer, d'oxyde de zinc et de stéarate de zinc, de silice et d'alumine et de silicone, de silice et d'alumine et de stéarate d'aluminium et de silicone ou d'alumine et de silicone.

14. Procédé selon la revendication 12, caractérisé par le fait qu'on utilise un nanopigment d'oxydes de titane et de cérium enrobés de silice.

15. Composition cosmétique non rincée pour cheveux, sous forme de gel, caractérisée par le fait qu'elle contient une quantité efficace pour protéger des cheveux contre les agressions atmosphériques et en particulier contre la lumière, comprise entre 0,2 et 2% en poids, d'au moins un nanopigment d'oxydes de métaux de diamètre moyen inférieur à 100 nanomètres choisis parmi le titane, le zinc, le cérium et le zirconium ou leurs mélanges, dans un support cosmétique gélifié.

16. Composition cosmétique selon la revendication 15, caractérisée par le fait qu'elle contient au moins un nanopigment de diamètre moyen compris entre 5 et 50 nanomètres.

17. Composition cosmétique selon la revendication 15 ou 16, caractérisée par le fait qu'elle contient un nanopigment d'oxyde de titane.

18. Composition cosmétique selon l'une quelconque des revendications 15 à 17, caractérisée par le fait qu'elle contient au moins un nanopigment d'oxydes métalliques non enrobés.

19. Composition cosmétique selon l'une quelconque des revendications 15 à 17, caractérisée par le fait qu'elle contient au moins un nanopigment d'oxydes métalliques enrobés ayant subi un ou plusieurs traitements de surface de nature chimique, électronique, mécanochimique et/ou mécanique avec des composés choisis parmi les aminoacides, la cire d'abeille, les acides gras, les alcools gras, les tensioactifs anioniques, les lécithines, les sels de sodium, potassium, zinc, fer ou aluminium d'acides gras, l'hexamétaphosphate de sodium, les alcoxydes métalliques, le polyéthylène, les silicones, les protéines, les alcanolamines et les oxydes de silicium.

20. Composition cosmétique selon la revendication 19, caractérisée par le fait qu'elle contient au moins un nanopigment d'oxydes métalliques enrobés choisis parmi les oxydes de titane enrobés de silice, de silice et d'oxyde de fer, de silice et d'alumine, d'alumine, d'alumine et de stéarate d'aluminium, d'alumine et de laurate d'aluminium, d'oxyde de fer et de stéarate de fer, d'oxyde de zinc et de stéarate de zinc, de silice et d'alumine et de silicone, de silice et d'alumine et de stéarate d'aluminium et de silicone, d'alumine et de silicone, de triéthanolamine, d'acide stéarique ou d'hexamétaphosphate de sodium.

21. Composition cosmétique selon la revendication 19, caractérisée par le fait qu'elle contient un mélange d'oxydes de titane et de cérium enrobés de silice.

22. Composition cosmétique selon l'une quelconque des revendications 15 à 21, caractérisée par le fait qu'elle contient 0.1 à 30% en poids, par rapport au poids total de la composition, d'agents gélifiants choisis parmi les acides polyacryliques réticulés par un agent polyfonctionnel, les dispersions aqueuses de copolymère d'acrylate d'ammonium/acrylamide réticulé par un agent de réticulation à polyinsaturation oléfinique, dispersé dans une émulsion eau-dans-l'huile constituée par 30% en poids dudit polymère, 25% en poids de paraffine, 4% de mélange de stéarate de sorbitan et de dérivé éthoxylé hydrophile et 41% en poids d'eau et les gels ayant une viscosité Epprecht-Drage, module 3, en solution à 1% dans l'eau à 25°C, supérieure ou égale à 0,50 Pa-s, résultant de l'interaction ionique d'un polymère cationique constitué par un copolymère de cellulose ou d'un dérivé de cellulose greffé avec un sel de monomère hydrosoluble d'ammonium quaternaire et d'un polymère anionique carboxylique ayant une viscosité capillaire absolue dans le diméthylformamide ou le méthanol, à une concentration de 5% et à 30°C, inférieure ou égale à 30 x 10⁻³ Pa.s.

23. Composition cosmétique selon la revendication 22, caractérisée par le fait qu'elle contient 0,2 à 10% en poids, par rapport au poids total de la composition, d'agents gélifiants.

24. Composition cosmétique selon l'une quelconque des revendications 15 à 23, caractérisée par le fait qu'elle contient un nanopigment d'oxyde de titane non enrobé de granulométrie comprise entre 15 et 40 nanomètres, dans un support cosmétique gélifié par un copolymère d'acide méthacrylique et de méthacrylate de méthyle associé à un copolymère d'hydroxyéthylcellulose greffé par voie radicalaire par du chlorure de diallyl diméthylammonium.

25. Composition cosmétique selon l'une quelconque des revendications 15 à 23, caractérisée par le fait qu'elle contient un nanopigment d'oxyde de titane non enrobé de granulométrie comprise entre 15 et 40 nanomètres, dans un support cosmétique gélifié par un copolymère d'acide méthacrylique et d'acrylate d'éthyle réticulé associé à un copolymère d'hydroxyéthylcellulose greffé par voie radicalaire par du chlorure de diallyl diméthylammonium.

26. Composition cosmétique selon l'une quelconque des revendications 15 à 25 caractérisée par le fait qu'elle contient en outre au moins un adjuvant cosmétique choisi parmi les polymères non- ioniques, anioniques ou cationiques, les conservateurs, les huiles, les agents de régulation du pH, les cires, les agents antigras, les agents séquestrants, les parfums, les colorants, les agents tensio-actifs cationiques, les protéines les silicones et les solvants organiques.

27. Composition cosmétique selon la revendication 26, caractérisée par le fait qu'elle contient des polymères non-ioniques choisis parmi les polymères ou copolymères de vinylpyrrolidone ou des polymères anioniques, en des concentrations comprises entre 0,1 et 5% en poids, par rapport au poids total de la composition.

## Claims

1. Use in an amount of between 0.2 and 2% by weight of metal oxide nanopigments of titanium, zinc, cerium or zirconium or mixtures thereof having an average diameter of less than 100 nanometers for protection of mechanical properties of hair against atmospheric attack, especially against light.

2. Use of nanopigments according to claim 1 wherein the diameter is between 5 and 50 nanometers.

3. Use of titanium oxide nanopigments in accordance with claim 1 or 2.

4. Use of non-coated metal oxide nanopigments in accordance with any one of claims 1 to 3.

5. Use of coated metal oxide nanopigments in accordance with any one of claims 1 to 3, the pigments having been treated by one or more chemical, electronic, mechanochemical and/or mechanical surface treatment techniques with compounds selected from amino acids, beeswax, fatty acids, fatty alcohols, anionic surfactants, lecithins, sodium, potassium, zinc, iron or aluminum salts of fatty acids, sodium hexametaphosphate, metallic alkoxides, polyethylene, silicones, proteins, alkanolamines and silicon oxides.

6. Use of coated metal oxide nanopigments according to claim 5 selected from titanium oxides coated with silica, silica and iron oxide, silica and alumina, alumina, alumina and aluminum stearate, alumina and aluminum laurate, iron oxide and iron stearate, zinc oxide and zinc stearate, silica, aluminum and silicone, silica, alumina, aluminium stearate and silicone, alumina and silicone, triethanolamine, stearic acid or sodium hexametaphosphate.

7. Use of titanium and cerium oxide nanopigments coated with silicon according to claim 5.

8. Method of protecting the mechanical properties of hair, particularly its tensile strength and elasticity, against atmospheric attack and in particular against light characterized in that it consists in applying to the hair an effective amount for protecting their mechanical properties, of between 0.2 and 2% by weight, of at least one metal oxide nanopigment of titanium, zinc, cerium or zirconium having a diameter of less than 100 nanometers in a cosmetic support, said application not being followed by rinsing.

9. Method according to claim 8 characterised in that at least one nanopigment having a diameter of between 5 and 50 nanometers is used.

10. Method according to claim 8 or 9 characterised in that a titanium oxide nanopigment is used.

11. Method according to any one of claims 8 to 10 characterised in that at least one uncoated metal oxide nanopigment is used.

12. Method according to any one of claims 8 to 10 characterised in that at least one coated metal oxide nanopigment is used which has been treated by one or more chemical, electronic, mechanochemical and/or mechanical surface treatment techniques with compounds selected from amino acids, beeswax, fatty acids, fatty alcohols, anionic surfactants, lecithins, sodium, potassium, zinc, iron or aluminum salts of fatty acids, sodium hexametaphosphate, metallic alkoxides, polyethylene, silicones, proteins, alkanolamines and silicon oxides.

13. Method according to claim 12 characterised in that at least one titanium oxide nanopigment is used which has been coated with silica, alumina, triethanolamine, stearic acid, sodium hexametaphosphate, silica and iron oxide, silica and alumina, alumina and aluminium stearate, alumina and aluminum laurate, iron oxide and iron stearate, zinc oxide and zinc stearate, silica, alumina and silicone, silica, alumina, aluminium stearate and silicone or alumina and silicone.

14. Method according to claim 12 characterised in that silica coated titanium and cerium oxides are used.

15. Non-rinse cosmetic composition for hair in the form of a gel characterised in that to protect mechanical properties of the hair against atmospheric attack, in particular against light, it contains an effective quantity of between 0.2 and 2% by weight of at least one metal oxide nanopigment of titanium, zinc, cerium or zirconium or mixtures thereof having a diameter of less than 100 nanometers, in a gelled cosmetic support.

16. Cosmetic composition according to claim 15 characterised in that it contains at least one nanopigment having an average diameter of between 5 and 50 nanometers.

17. Cosmetic composition according to claim 15 or 16 characterised in that it contains a titanium oxide nanopigment.

18. Cosmetic composition according to any one of claims 15 to 17 characterised in that it contains at least one uncoated metal oxide nanopigment.

19. Cosmetic composition according to any one of claims 15 to 17 characterised in that it contains at least one coated metal oxide nanopigment which has been treated by one or more chemical, electronic, mechanochemical and/or mechanical surface treatment techniques with compounds selected from amino acids, beeswax, fatty acids, fatty alcohols, anionic surfactants, lecithins, sodium, potassium, zinc, iron or aluminum salts of fatty acids, sodium hexametaphosphate, metallic alkoxides, polyethylene, silicones, proteins, alkanolamines and silicon oxides.

20. Cosmetic composition according to claim 19 characterised in that it contains at least one coated metal oxide nanopigment selected from titanium oxides coated with silica, silica and iron oxide, silica and alumina, alumina, alumina and aluminum stearate, alumina and aluminum laurate; iron oxide and iron stearate, zinc oxide and zinc stearate, silica, aluminum and silicone, silica, alumina, aluminium stearate and silicone, alumina and silicone, triethanolamine, stearic acid and sodium hexametaphosphate.

21. Cosmetic composition according to claim 19 characterised in that it contains a mixture of silica coated titanium and cerium oxides.

22. Cosmetic composition according to any one of claims 15 to 21 characterised in that it contains 0.1 to 30% by weight with respect to the total composition weight of gelling agents selected from polyacrylic acids cross linked using a polyfunctional agent, aqueous dispersions of ammonium acrylate/acrylamide copolymer cross linked using a polyunsaturated olefinic reticulating agent, dispersed in a water-in-oil emulsion constituted by 30% by weight of said polymer, 25% by weight of paraffin, 4% of a mixture of sorbitan stearate and a hydrophilic ethoxylated derivative and 41% by weight of water, such gels having a modulus 3 Epprecht-Drage viscosity in 1% solution in water at 25°C greater than or equal to 0.50 Pa.s, the gels resulting from the ionic interaction of a cationic polymer constituted by a copolymer of cellulose or a cellulose derivative grafted with a hydrosoluble quaternary ammonium salt monomer and an anionic carboxylic polymer having an absolute capillary viscosity in dimethylformamide or methanol at a concentration of 5% and at 30°C less than or equal to 30 x 10⁻³ Pa.s.

23. Cosmetic composition according to claim 22 characterised in that it contains 0.2 to 10% by weight with respect to the total composition weight of gelling agents.

24. Cosmetic composition according to any one of claims 15 to 23 characterised in that it contains an uncoated titanium oxide nanopigment with a granulometry of between 15 and 40 nanometers in a cosmetic support gelled with a copolymer of methacrylic acid and methyl methacrylate associated with a copolymer of hydroxyethylcellulose grafted by a radical mechanism with diallyl dimethylammonium chloride.

25. Cosmetic composition according to any one of claims 15 to 23 characterised in that it contains an uncoated titanium oxide nanopigment with a granulometry of between 15 and 40 nanometers in a cosmetic support gelled by a cross linked copolymer of methacrylic acid and ethyl acrylate associated with a copolymer of hydroxyethylcellulosed grafted by a radical mechanism with diallyl dimethylammonium chloride.

26. Cosmetic composition according to any one of claims 15 to 25 characterised in that it further contains at least one cosmetic additive selected from non-ionic, anionic or cationic polymers, preservatives, oils, pH regulators, waxes, antifatting agents, sequestrating agents, perfumes, dyes, cationic surfactants, proteins, silicones and organic solvents.

27. Cosmetic composition according to claim 26 characterised in that it contains non-ionic polymers selected from polymers or copolymers of vinylpyrrolidone or anionic polymers in concentrations of between 0.1 and 5% by weight with respect to the total composition weight.

## Patentansprüche

1. Verwendung in einer Menge von 0,2 bis 2 Gew.% von Nanopigmenten aus Oxiden von Metallen, ausgewählt aus Titan, Zink, Cer und Zirkon, oder aus deren Mischungen, mit einem mittleren Durchmesser unterhalb 100 nm, als Mittel zum Schutz der mechanischen Eigenschaften der Haare vor agressiven Einwirkungen aus der Atmosphäre, insbesondere vor Licht.

2. Verwendung gemäß Anspruch 1 von Nanopigmenten eines Durchmessers von 5 bis 50 nm.

3. Verwendung gemäß Anspruch 1 oder 2 von Nanopigmenten aus Titanoxiden.

4. Verwendung gemäß jedem Anspruch 1 bis 3 von Nanopigmenten aus Metalloxiden, welche nicht umhüllt sind.

5. Verwendung gemäß jedem Anspruch 1 bis 3 von Nanopigmenten aus umhüllten Metalloxiden, wobei diese Pigmente einer oder mehreren Oberflächenbehandlungen chemischer, elektronischer, mechanochemischer und/oder mechanischer Art mit Verbindungen unterzogen worden sind, die aus Aminosäuren, Bienenwachs, Fettsäuren, Fettalkoholen, anionischen oberflächenaktiven Mitteln, Lecithinen, Natrium-, Kalium-, Zink-, Eisen- oder Aluminiumsalzen von Fettsäuren, Natriumhexametaphosphat, Metallalkoxiden, Polyethylen, Silikonen, Proteinen, Alkanolaminen und Siliziumoxiden ausgewählt sind.

6. Verwendung gemäß Anspruch 5 von Nanopigmenten aus umhüllten Metalloxiden, ausgewählt aus Titanoxiden, die mit Siliziumdioxid, Siliziumdioxid und Eisenoxid, Siliziumdioxid und Aluminiumoxid, Aluminiumoxid, Aluminiumoxid und Aluminiumstearat, Aluminiumoxid und Aluminiumlaurat, Eisenoxid und Eisenstearat, Zinkoxid und Zinkstearat, Siliziumdioxid und Aluminiumoxid und Silikon, Siliziumidioxid und Aluminiumoxid und Aluminiumstearat und Silikon, Aluminiumoxid und Silikon, Triethanolamin, Stearinsäure oder Natriumhexametaphosphat umhüllt sind.

7. Verwendung gemäß Anspruch 5 von Nanopigmenten aus mit Siliziumdioxid umhüllten Titan- und Ceroxiden.

8. Verfahren zum Schutz der mechanischen Eigenschaften der Haare, und insbesondere von deren Widerstandsfähigkeit gegenüber Traktion und von deren Elastizität, vor agressiven Einwirkungen aus der Atmosphäre und insbesondere vor Licht,
dadurch **gekennzeichnet**, daß
das Verfahren darauf beruht, daß man auf die Haare eine zum Schutz ihrer mechanischen Eigenschaften wirksame Menge von 0,2 bis 2 Gew.% mindestens eines Nanopigments eines mittleren Durchmessers unterhalb 100 nm aus Oxiden von Metallen, ausgewählt aus Titan, Zink, Cer und Zirkon, in einem kosmetischen Trägermittel aufbringt, wobei nach dieser Aufbringung keine Spülung erfolgt.

9. Verfahren gemäß Anspruch 8,
dadurch **gekennzeichnet**, daß
man mindestens ein Nanopigment eines Durchmessers von 5 bis 50 nm verwendet.

10. Verfahren gemäß Anpruch 8 oder 9,
dadurch **gekennzeichnet**, daß
man ein Nanopigment aus Titanoxid verwendet.

11. Verfahren gemäß jedem Anspruch 8 bis 10,
dadurch **gekennzeichnet**, daß
man mindestens ein Nanopigment aus nicht umhüllten Metalloxiden verwendet.

12. Verfahren gemäß jedem Anspruch 8 bis 10,
dadurch **gekennzeichnet**, daß
man mindestens ein Nanopigment aus umhüllten Metalloxiden verwendet, welche einer oder mehreren Oberflächenbehandlungen chemischer, elektronischer, mechanochemischer und/oder mechanischer Art mit Verbindungen unterzogen worden sind, die aus Aminosäuren, Bienenwachs, Fettsäuren, Fettalkoholen, anionischen oberflächenaktiven Mitteln, Lecithinen, Natrium-, Kalium-, Zink-, Eisen- oder Aluminiumsalzen von Fettsäuren, Natriumhexametaphosphat, Metallalkoxiden, Polyethylen, Silikonen, Proteinen, Alkanolaminen und Siliziumoxiden ausgewählt sind.

13. Verfahren gemäß Anspruch 12,
dadurch **gekennzeichnet**, daß
man mindestens ein Nanopigment aus Titanoxiden verwendet, die mit Siliziumdioxid, Aluminiumoxid, Triethanolamin, Stearinsäure, Natriumhexametaphosphat, Siliziumdioxid und Eisenoxid, Siliziumdioxid und Aluminiumoxid, Aluminiumoxid und Aluminiumstearat, Aluminiumoxid und Aluminiumlaurat, Eisenoxid und Eisenstearat, Zinkoxid und Zinkstearat, Siliziumdioxid und Aluminiumoxid und Silikon, Siliziumdioxid und Aluminiumoxid und Aluminiumstearat und Silikon oder mit Aluminiumoxid und Silikon umhüllt sind.

14. Verfahren gemäß Anpruch 12,
dadurch **gekennzeichnet**, daß
man ein Nanopigment aus mit Siliziumdioxid umhüllten Titan- und Ceroxiden verwendet.

15. Kosmetische Zusammensetzung, die nicht zur Spülung vorgesehen ist, für Haare, in Form eines Gels,
dadurch **gekennzeichnet**, daß
sie eine zum Schutz der mechanischen Eigenschaften der Haare vor agressiven Einwirkungen aus der Atmosphäre und insbesondere vor Licht wirksame Menge von 0,2 bis 2 Gew.% mindestens eines Nanopigments eines mittleren Durchmessers unterhalb 100 nm aus Oxiden von Metallen, ausgewählt aus Titan, Zink, Cer und Zirkon, oder aus deren Mischungen in einem gelierten kosmetischen Trägermittel enthält.

16. Kosmetische Zusammensetzung gemäß Anspruch 15,
dadurch **gekennzeichnet**, daß
sie mindestens ein Nanopigment eines mittleren Durchmessers von 5 bis 50 nm enthält.

17. Kosmetische Zusammensetzung gemäß Anspruch 15 oder 16,
dadurch **gekennzeichnet**, daß
sie ein Nanopigment aus Titanoxid enthält.

18. Kosmetische Zusammensetzung gemäß jedem Anspruch 15 bis 17,
dadurch **gekennzeichnet**, daß
sie mindestens ein Nanopigment aus nicht umhüllten Metalloxiden enthält.

19. Kosmetische Zusammensetzung gemäß jedem Anspruch 15 bis 17,
dadurch **gekennzeichnet**, daß
sie mindestens ein Nanopigment aus umhüllten Metalloxiden enthält, die einer oder mehreren Oberflächenbehandlungen chemischer, elektronischer, mechanochemischer oder mechanischer Art mit Verbindungen unterzogen worden sind, die aus Aminosäuren, Bienenwachs, Fettsäuren, Fettalkoholen, anionischen oberflächenaktiven Mitteln, Lecithinen, Natrium-, Kalium-, Zink-, Eisen- oder Aluminiumsalzen von Fettsäuren, Natriumhexametaphosphat, Metallalkoxiden, Polyethylen, Silikonen, Proteinen, Alkanolaminen und Siliziumoxiden ausgewählt sind.

20. Kosmetische Zusammensetzung gemäß Anspruch 19,
dadurch **gekennzeichnet**, daß
sie mindestens ein Nanopigment aus umhüllten Metalloxiden enthält, ausgewählt aus Titanoxiden, die mit Siliziumdioxid, Siliziumdioxid und Eisenoxid, Siliziumdioxid und Aluminiumoxid, Aluminiumoxid, Aluminiumoxid und Aluminiumstearat, Aluminiumoxid und Aluminiumlaurat, Eisenoxid und Eisenstearat, Zinkoxid und Zinkstearat, Siliziumdioxid und Aluminiumoxid und Silikon, Siliziumidioxid und Aluminiumoxid und Aluminiumstearat und Silikon, Aluminiumoxid und Silikon, Triethanolamin, Stearinsäure oder Natriumhexametaphosphat umhüllt sind.

21. Kosmetische Zusammensetzung gemäß Anspruch 19,
dadurch **gekennzeichnet**, daß
sie eine Mischung aus mit Siliziumdioxid umhüllten Titan- und Ceroxiden enthält.

22. Kosmetische Zusammensetzung gemäß jedem Anspruch 15 bis 21,
dadurch **gekennzeichnet**, daß
sie 0,1 bis 30 Gew.%, bezogen auf das Gesamtgewicht der Zusammensetzung, Geliermittel enthält, ausgewählt aus mit einem polyfunktionellen Mittel vernetzten Polyacrylsäuren, wässrigen Dispersionen eines Copolymers aus Ammoniumacrylat/Acrylamid, das mit einem Vernetzungsmittel mit olefinischer Mehrfachungesättigtheit vernetzt ist und in einer Wasser-in-Öl-Emulsion dispergiert vorliegt, die aus 30 Gew.% des genannten Polymers, 25 Gew.% Paraffin, 4 Gew.% einer Mischung aus Sorbitanstearat und hydrophilem ethoxylierten Derivat davon sowie aus 41 Gew.% Wasser zusammengesetzt ist, und aus Gelen mit einer Epprecht-Drage-Viskosität, Modul 3, in Lösung von 1% in Wasser bei 25°C, oberhalb oder gleich 0,50 Pa x s, welche sich aus der ionischen Wechselwirkung eines kationischen Polymers aus einem Copolymer von Cellulose oder einem Cellulosederivat, welche mit einem Salz eines wasserlöslichen Monomers einer quaternären Ammoniumverbindung gepfropft sind, und eines anionischen Carboxylpolymers ergeben, das eine absolute Kapillarviskosität in Dimethylformamid oder Methanol in einer Konzentration von 5% und bei 30°C von unterhalb oder gleich 30 x 10⁻³ Pa x s aufweist.

23. Kosmetische Zusammensetzung gemäß Anspruch 22,
dadurch **gekennzeichnet**, daß
sie 0,2 bis 10 Gew.%, bezogen auf das Gesamtgewicht der Zusammensetzung, Geliermittel enthält.

24. Kosmetische Zusammensetzung gemäß jedem Anspruch 15 bis 23,
dadurch **gekennzeichnet**, daß
sie ein Nanopigment aus nicht umhülltem Titanoxid einer Korngröße von 15 bis 40 nm in einem kosmetischen Trägermittel enthält, das durch ein Copolymer aus Methacrylsäure und Methylmethacrylat geliert ist, das mit einem Copolymer von Hydroxyethylcellulose assoziiert ist, welches radikalisch mit Dialllyldimethylammoniumchlorid gepfropft ist.

25. Kosmetische Zusammensetzung gemäß jedem Anspruch 15 bis 23,
dadurch **gekennzeichnet**, daß
sie ein Nanopigment aus nicht umhülltem Titanoxid einer Korngröße von 15 bis 40 nm in einem kosmetischen Trägermittel enthält, das durch ein vernetztes Copolymer aus Methacrylsäure und Ethylacrylat geliert ist, das mit einem Copolymer von Hydroxyethylcellulose assoziiert ist, welches radikalisch mit Diallyldimethylammoniumchlorid gepfropft ist.

26. Kosmetische Zusammensetzung gemäß jedem Anspruch 15 bis 25,
dadurch **gekennzeichnet**, daß
sie ausserdem mindestens ein kosmetisches Hilfsmittel enthält, ausgewählt aus nicht-ionischen, anionischen oder kationischen Polymeren, Konservierungsstoffen, Ölen, Regulierungsmitteln des pH, Wachsen, Antifettmitteln, Sequestriermitteln, Parfüm-Produkten, Farbstoffen, kationischen, oberflächenaktiven Mitteln, Proteinen, Silikonen und aus organischen Lösungsmitteln.

27. Kosmetische Zusammensetzung gemäß Anspruch 26,
dadurch **gekennzeichnet**, daß
sie nicht-ionische Polymere, ausgewählt aus Polymeren und Copolymeren von Vinylpyrrolidon, oder anionische Polymere in Konzentrationen von 0,1 bis 5 Gew.%, bezogen auf das Gesamtgewicht der Zusammensetzung, enthält.
